# EUROPEAN PATENT APPLICATION

(11) **EP 0 627 223 A1**
(43) Date of publication of application: **07.12.1994**
(21) Application number: 94107936.0
(22) Date of filing: 24.05.1994
(51) Int. Cl.: A61K 37/02, A61K 35/78

(54) **Topical composition with therapeutic and cosmetic activity, containing aloe vera and modified natural collagen**

(30) Priority: 04.06.1993 IT MI931187
(71) Applicant: SAVALON S.r.l., I-20124 Milano (IT)
(72) Inventor: Donati, Claudio, I-40100 Boplogna (IT)
(74) Representative: Frignoli, Luigi

(57) **Abstract**

The invention relates to a composition for topical administration of pharmacologically active or cosmetic substances in which said substances are present in an emulsion comprising aloe vera gel and modified natural collagen the amino acids of which are free because of breakage of the covalent dehydrolysine-norleucine bonds between the polypeptide chains.

The said emulsion of aloe vera and modified natural collagen itself possesses antiphlogistic and cicatrizant therapeutic properties.

## Description

This invention relates to a composition comprising, as essential components, an emulsion of aloe vera gel and modified natural collagen usable both for topical administration of pharmacologically active or cosmetic substances, and as such for dermatological therapeutic use as an antiphlogistic or cicatrizant.

It is known to use in the cosmetic and therapeutic field lyophilized heterologous sheets and hydrolyzed solutions of natural collagens (of animal origin), used together with pharmacologically active principles (such as hyaluronic acid) or with the common ingredients of cosmetic products.

For example, sterilized lyophilized heterologous collagen is used topically in surgery to facilitate the treatment of many dermic lesions (such as deep burns, bed sores, varicose ulcers) and as a hemostatic in dentistry. Lyophilized collagen is mainly used for its absorbent and hemostatic power.

Lyophilized collagen sheets or plates can be enriched with the most varied active principles which exhibit their activity (once applied to the cutis) practically without any direct influence of the collagen.

Hydrolyzed solutions of natural collagens are mainly used in cosmetics.

It is well known that collagen is a scleroprotein forming the main component of fibrous connective tissue. It is present in animal products such as tendons, cartilage, skin etc. Collagen consists of polypeptide chains formed from amino acid residues with peptide bonds.

According to Rich and Crickm, each polypeptide chain is arranged as a spiral, the chains being twisted together in threes with opposite twist, forming a superposed spiral. They can be linked transversely by hydrogen bridges.

Collagen is insoluble in organic solvents, in water and in dilute acid and alkaline solutions at ordinary temperature, in which it absorbs water and undergoes characteristic swelling.

When heated in water to a determined temperature (contraction temperature) the collagen fibres undergo irreversible contraction, explained by the fact that the polypeptide chains diverge because water molecules are introduced between them, causing their radical modification.

If the heating in water is prolonged above the contraction temperature the collagen is transformed into gelatin.

The present invention relates to a composition comprising collagen which has been mechanically modified by emulsion together with aloe vera. Aloe vera is itself a fundamental component in that its presence is decisive for preparing the modified collagen.

The process for preparing the composition of the invention comprises essentially the following steps:
1) Preparing a first emulsion of "inverse mixture" type (ie in the form of water droplets surrounded by surfactant molecules) by mixing oil and/or fatty substances with aqueous solutions of natural collagen and aloe vera gel.
2) Preparing a first emulsion of "direct mixture" type (ie in the form of oil droplets surrounded by molecules of various types) by mixing oil and/or fatty substances with solubilizers, to which an aqueous solution is added comprising further natural collagen in aqueous solution and aloe vera gel; pharmacologically active principles can be added to this second solution.
3) Mixing together the aforesaid first and second emulsion in a microemulsifying reactor to obtain a final microemulsion containing modified collagen for topical therapeutic or cosmetic uses.

Proceeding in the aforesaid manner, the modification of the collagen is achieved by mechanically breaking the macromolecule followed by transforming the particles in a like number of minuscule reactors which interchange ions of various types when the two emulsions are mixed together in the microemulsifying reactor.

It is important to note that the collagen can be also modified in another manner, for example by hydrolysis. In this case there is an authentic proteolysis process in which the amino acids are transformed into denatured proteins, and as such, because of their molecular weight, not only do they not penetrate into the cutis but behave as foreign bodies.

According to the present invention it has been surprisingly found that the mechanically modified collagen is no longer limited to performing its traditional mechanical functions, but exhibits a synergic action with respect to the active principles microemulsified with it, as the amino acids of the collagen are intact and active by being free (in this respect the covalent dehydrolysinenorleucine bonds between the polypeptide chains are broken) and can hence penetrate through the cutis, the mucosa on which the emulsion is applied performing a very important function as an active vehicle for the pharmacological or cosmetic substance to be topically applied.

The emulsion obtained has a high capacity for solubilizing or carrying medicaments, for example numerous antibiotics (such as gramicidin, which has affinity with natural collagen), related to the properties of the system.

It has been found that preparations for topical use of any type containing collagen and aloe vera emulsified together have much better transcutaneous carrying properties than those with the same components but with unmodified collagen.

A study conducted on two groups of patients suffering from bed sores, wounds and burns, one treated with lyophilized heterologous collagen and the other with collagen modified according to the present invention, showed a significant speed of recovery in favour of the modified collagen (30% reduction in recovery time).

The following examples illustrate the procedure for preparing the modified collagen of the present invention and the use of the preparation as a vehicle for pharmacologically active substances.

### EXAMPLE 1

Between 5 g and 12 g (preferably 8 g) of Cremophor S9, between 1 g and 3.6 g (preferably 2 g) of Cremaphor RH40 and between 1 g and 3 g (preferably 2 g) of hydroxyethyl cetylammonium phosphate are mixed together in a turboemulsifier temperature-controlled at 60°C.

A solution composed of 17 g (± 2 g) of aloe vera gel, 11 g (± 2 g) of a natural collagen solution and sufficient distilled water to make up to 100 g is slowly added to this fatty phase. After 60 minutes of agitation at a speed of 3600 rpm a first microemulsion of "inverse mixture" type is obtained.

Between 4 g and 8 g (preferably 6 g) of Cremophor WO7 and between 5 g and 8 g (preferably 7 g) of Luvitol EHO are mixed together in a second turboemulsifier identical to the first, temperature-controlled at 60°C. To this is added a solution of 7.6 g (± 2 g) of natural collagen, 28 g (± 3 g) of aloe vera and sufficient distilled water to make up to 100 g. A second microemulsion of "direct type" is obtained.

100 g of the first microemulsion and 100 g of the second microemulsion are then mixed together in the turboemulsifier at a speed exceeding 3800 rpm for a period of not less than 60 minutes at a temperature of 35°C.

Finally 0.15 g of alginic acid in water and 0.65 g of triethanolamine are added to thicken the mass obtained.

The composition obtained in this manner has itself cicatrizant properties. In this respect it facilitates the healing of many dermic lesions such as deep burns, bed sores, varicose ulcers and the like.

It should be noted that other components can be added to the first emulsion depending on the characteristics required of the final composition.

### EXAMPLE 2

The procedure of Example 1 is followed but with the difference that 0.2 g of vitamin B2 and 1 g of bovine bile salts are added to the first emulsion as a replacement for an equivalent quantity of water.

The composition obtained in this manner can be used as an anticellulitic.

### EXAMPLE 3

7.5 g of PEG-9 stearate, 1.2 g of PEG-7 hydrogenated castor oil, 0.8 g of alpha-bisabolol, 12 g of cetearyl octanoate, 1 g of vitamin A and D and 1 g of 2-amino-n-butyl alcohol are mixed together in a turboemulsifier temperature-controlled at 60°C.

A solution composed of 37.5 g of aloe vera gel, 12.5 g of natural collagen solution, 4 g of allantoin, 1 g of hyaluronic acid and 22.5 g of distilled water is slowly added to this fatty phase. After 50 minutes of agitation at a speed of 3800 rpm 100 g of a first microemulsion of "inverse mixture" type are obtained.

2.5 g of ceteareth-6 with stearyl alcohol, 0.6 g of raceme alpha-bisabolol and 2.5 g of wild rose oil are mixed together in a second turboemulsifier identical to the first, temperature controlled at 60-70°C. A solution corresponding to 7.5 g of natural collagen, 0.5 g of polyquaternary 16, 2.4 g of raceme alpha-bisabolol, 1 g of vitamin B6, 28.5 g of aloe vera and 49 g of distilled water are added thereto to obtain a second microemulsion of "direct mixture" type.

25 g of the first microemulsion and 25 g of the second microemulsion are then mixed together in the turboemulsifier at a speed exceeding 3800 rpm for a period of not less than 60 minutes at a temperature of 35°C.

Finally 50 g of a 0.6% solution of alginic acid in water are added to obtain after 60 minutes a thick gelatin of pH 5.8..

The composition obtained in this manner has antiphlogistic, anesthetic, antihistaminic and cicatrizant properties.

## Claims

1. A composition with therapeutic and cosmetic activity for topical use, characterised by comprising an emulsion of aloe vera and modified natural collagen the amino acids of which are free because of breakage of the covalent dehydrolysine-norleucine bonds between the polypeptide chains, the aloe vera:collagen weight ratio being within the range 1.7-2.3:0.7-1.3.

2. A composition as claimed in claim 1, characterised in that the aloe vera:collagen weight ratio is 2:1.

3. A composition as claimed in claims 1 and 2, characterised in that said emulsion comprises substances with pharmacological and/or cosmetic activity.

4. A composition as claimed in claim 3, characterised in that said substances are chosen from antibiotics, hyaluronic acid, alginic acid, allantoin, wild rose oil, vitamins, alpha-bisabolol and bovine bile salts.

5. A composition as claimed in claims 1 to 4, characterised by being applied in the form of a thin layer on a support of textile nature.

6. A method for preparing the compositions of claims 1 to 4, characterised by preparing a first emulsion of "inverse mixture" type by mixing oils and/or fatty substances with an aqueous solution of natural collagen and aloe vera gel, and a second emulsion of "direct mixture" type by mixing oils and/or fatty substances with solubilizers to which an aqueous solution comprising natural collagen in aqueous solution and aloe vera gel is added, said first and second emulsion being then mixed together in a microemulsifying reactor.

7. A method as claimed in claim 6, characterised in that said pharmacologically active substances are added to said second emulsion.
